# EUROPEAN PATENT SPECIFICATION

(11) **EP 2 066 253 B1**
(45) Date of publication and mention of the grant of the patent: **16.07.2014**
(21) Application number: 07843620.1
(22) Date of filing: 01.10.2007
(51) Int. Cl.: A61B 18/20, A61N 5/067, H01S 3/094, H01S 3/16

(54) **TREATMENT OF SKIN BY A SOLID-STATE LASER**
BEHANDLUNG DER HAUT DURCH EINEN FESTKÖRPERLASER
TRAITEMENT DE LA PEAU PAR UN LASER À L'ÉTAT SOLIDE

(30) Priority: 29.09.2006 US 848083 P
(43) Date of publication of application: 10.06.2009
(60) Divisional application: 11172575.0
(73) Proprietor: CANDELA CORPORATION, Wayland, MA 01778 (US)
(72) Inventor: JONES, Christopher, J., Leicester, MA 01524 (US); BHAWALKAR, Jayant, D., Brighton, MA 02135 (US)
(74) Representative: Lawrence, John
(86) International application number: PCT/US2007/080093
(87) International publication number: WO 2008/042854

(56) References cited:
- EP-A- 0 284 330
- WO-A-99/16366
- DE-U1- 9 305 325
- US-A- 4 965 803
- US-A- 5 757 839
- US-A- 5 793 791
- US-A- 6 122 097
- US-B1- 6 358 243
- US-B1- 6 539 041

## Description

### FIELD OF THE INVENTION

The invention relates generally to a device for skin treatment using radiation. More particularly, the invention relates to a device including a first solid state transition metal laser exciting a second solid state laser for treatment of skin, such as hair removal, pigmented lesions, tattoos, vascular lesions, wrinkles, acne, skin tightening, and/or fat reduction.

### BACKGROUND OF THE INVENTION

Lasers are widely used in dermatological applications such as hair removal, removal of pigmented lesions, tattoos, vascular lesions, wrinkles, acne, and skin tightening. Dermatological laser treatments are typically based on selective targeting of a chromophore in the skin by an appropriate choice of wavelength and pulse duration of the laser light. Although lasers can provide better results than most other light sources, most medical laser devices use only a single wavelength of light. This limits the range of applications for which a particular medical laser can be used. Therefore, several different lasers can be needed to treat more than one skin condition.

In addition, solid-state lasers are typically pumped by flashlamps to get the large pulse energies required for creating the desired thermal profile in the skin. While such lasers can provide large output energies, the beam quality is generally poor and frequency conversion can be difficult.

DE 93 05 325 Ul discloses a dental laser with an Alexandrite laser or Ti:Sapphire laser arranged to pump laser crystal provided in a handpiece.

US 4 955 803 discloses a room temperature, laser-pumped, q-switched, thalium-doped solid state laser for producing laser pulses of 2 microns.

US 5 757 839 discloses apparatus for producing high repetition rate, high power laser pulses whilst avoiding thermal distortion.

US 6 539 041 discloses apparatus comprising a solid state dye laser for generating laser pump energy, a passive Q-switch for generating high intensity bursts of laser pump energy, a frequency doubler for generating dye laser pump energy and a solid state laser dye element for generating optical energy output.

### SUMMARY OF THE INVENTION

The invention is defined in the appended set of claims. Methods mentioned herein, apart from tattoo removal and non-therapeutic hair removal, do not form part of the invention.

Transition metal solid state lasers can be used to excite another laser. Multi-laser systems based on laser pumping of one or more other lasers can provide two or more wavelengths with little additional cost, complexity or size. Also, a laser pumped laser generally can have better beam quality than a flashlamp pumped laser. Better beam quality can permit the generation of additional wavelengths by various methods of non-linear frequency conversion. Furthermore, laser pumping is particularly advantageous for lasers that are difficult to pump with other conventional sources like laser diodes and flashlamps.

Excitation of one laser by another enables generation of a new wavelength, along with an increase in brightness, which further allows non-linear frequency conversion. The increase in brightness can also allow the beam to be focused to a small spot of high intensity laser energy that can be used to cut tissue in surgical applications.

In other examples, any of the aspects above or any apparatus or method described herein can include one or more of the following features. In one embodiment, treating the first condition can include removing black tattoos. In various embodiments, the laser system can further include optical lenses. The optical lenses can be used to direct a first part of the first output beam to a second solid-state laser. The optical fiber is used to direct the first part of the first output beam to the second solid-state laser. The laser system further includes a handpiece. The handpiece is used to direct a first part of the second output beam to the target region. The second condition can include removing violet tattoos, blue tattoos, green tattoos, black tattoos, or any combination thereof.

In other examples, any of the aspects above or any apparatus or method described herein can include one or more of the following features. In various embodiments, the laser system can further include an output coupler mirror. The output coupler mirror can form a dual wavelength output beam from a second part of the first output beam that passes through the second solid- state laser and laser radiation from the second solid-state laser. In one embodiment, the method can further include directing the dual wavelength output beam to the target region to treat the first condition and a second condition. In various embodiments, the method can further include generating, based on laser radiation received from the second solid-state laser, the second output beam using a nonlinear frequency converter. The laser system can include the nonlinear frequency converter. Treating the third condition can include removing red tattoos, orange tattoos, yellow tattoos, or any combination thereof. In some embodiments, the laser system can further include a q-switching element in the first solid-state laser to generate high peak power pulses of the first output beam.

In other examples, any of the aspects above or any apparatus or method described herein can include one or more of the following features. In various embodiments, the laser system can further include optical lenses adapted to receive laser radiation from the first solid-state laser for directing the first part of the first output beam to the second solid-state laser. The delivery device includes a handpiece, and the second solid state laser can be housed in the handpiece. The delivery device can include an output coupler mirror for forming a dual wavelength output beam from a second part of the first output beam that passes through the second solid-state laser and laser radiation from the second solid-state laser. In some embodiments, the laser system can further include a nonlinear frequency converter for generating, based on laser radiation received from the second solid-state laser, the second output beam. The laser system can further include a q-switching element in the first solid-state laser for generating high peak power pulses of the first output beam. The second solid state laser includes a rare-earth doped gain medium. The rare earth doped gain medium can include: YAG, YAP, YVO4, YLF, YSGG, GSGG, FAP, GdVO₄, KGd(WO₄)₂, SFAP, glass, ceramic, or any combination thereof. The rare-earth doped gain medium can be doped with rare-earth ions. Rare-earth ions can include: Nd, Yb, Er, Ho, Tm, Sm, Ce, or any combination thereof.

Also disclosed herein is a laser system for tattoo removal including a Q- switched alexandrite laser for producing a first output beam, a Nd:YAG laser for producing a second output beam, and a delivery device for directing the second output beam to a target region of skin. The wavelength of the first output beam is about 755 nm. The wavelength of the second output beam is about 1064 nm. The Nd:YAG laser is adapted to absorb the first output beam for generating excitation to produce the second output beam. The second output beam is used for removing black tattoos.

Also disclosed herein is a laser system for tattoo removal including a Q- switched alexandrite laser for producing a first output beam, a Nd: YAG laser for producing a second output beam, a KTP crystal for generating a third output beam, and a delivery device for directing the third output beam to a target region of skin. The wavelength of the first output beam is about 755 nm. The wavelength of the second output beam is about 1064 nm. The Nd:YAG laser is adapted to absorb the first output beam for generating excitation to produce the second output beam. The KTP crystal is adapted to absorb the second output beam for generating the third output beam. The wavelength of the third output beam is about 532 nm. The third output beam is used for removing red tattoos, orange tattoos, yellow tattoos, or any combination of tattoos.

In other examples, any of the aspects above or any apparatus or method described herein can include one or more of the following features. In various embodiments, the laser system can further include a nonlinear frequency converter for generating a third output beam from at least a part of the second output beam. The nonlinear frequency converter can be a second harmonic generation converter, a third harmonic generation converter, a fourth harmonic generation converter, an optical parametric oscillator, or a Raman shifting converter. In some embodiments, the first solid-state laser can include a first host material. The second solid-state laser can include a second host material. The second host material can include a crystalline structure. The crystalline structure can include: YAG, YAP, YVO4, YLF, YSGG, GSGG, FAP, GdVO₄, KGd(WO₄)₂, or SFAP. The second host material can include an amorphous structure. The amorphous structure can include: glass or ceramic YAG. The second host material is doped with a rare-earth ion selected from: Nd, Yb, Er, Ho or Tm. The doping of the second host material can include a rare-earth ion selected from: Nd, Yb, Er, Ho or Tm. The doping of the second host crystal can include co-doping with one or more ions selected from: Cr, Nd, Yb, Er, Ho or Tm. The rare-earth doped gain medium can be Nd: YAG.

In other examples, any of the aspects above or any apparatus or method described herein can include one or more of the following features. The second output beam can include a single pulse or a train of pulses, each pulse of duration between approximately 1 ns and approximately 500 ms. Each pulse can have an energy between about 1 microJoule and about 100 Joules. The first beam output can include a wavelength between about 400 nm and about 1000 nm. The second beam output can include a wavelength between about 400 nm and about 3000 nm.

The system may include a flashlamp, a first gain medium excited by the flashlamp for producing a first output beam, a second gain medium excited by a part of the first output beam for producing a second output beam, a first coupling element for coupling the part of the first output beam to the second gain medium, and a second coupling element for coupling a part of the second output beam out of a cavity containing the second gain medium.

The system may include a flashlamp pumped Alexandrite laser producing a first output beam having a first wavelength and a first beam path, and an Alexandrite-pumped neodymium laser. The Alexandrite-pumped neodymium laser is movable from a first position not in the first beam path to a second position in said first beam path. The Alexandrite-pumped neodymium laser is also capable of absorbing at least some of the first output beam and producing a second output beam having a second wavelength and a second beam path coaxial with the first beam path. The neodymium laser includes a neodymium doped laser gain material.

In other examples, any of the aspects above or any apparatus or method described herein can include one or more of the following features. In various embodiments, the Alexandrite laser can include a KD*P q-switch, producing high peak power pulses. The laser system can further include a KTP second harmonic generator movable from a first position not in the second beam path to a position in the second beam path producing a third output beam having a third output wavelength and a third beam path coaxial with the second beam path. The neodymium laser can further include a Cr⁴⁺:YAG passive q-switch where the second output beam comprises a train of high peak power pulses. The laser system can further include a KTP second-harmonic generator movable from a first position not in the second beam path to a position in the second beam path producing a third output beam comprising a train of high peak power pulses having a third output wavelength and a third beam path coaxial with the second beam path.

In other examples, any of the aspects above or any apparatus or method described herein can include one or more of the following features. In various embodiments, the Alexandrite laser can include a KD*P q-switch, producing high peak power pulses. The handpiece can further include a KTP second-harmonic generator positioned in the second beam path, producing third output beam having a third wavelength. The neodymium laser can further include a Cr⁴⁺:YAG passive q-switch producing a train of high peak power pulses, where the handpiece further comprises a KTP second-harmonic generator positioned in the second beam path, producing a third output beam having a third wavelength.

Advantages of the invention include one or more of the following. Techniques to generate new wavelengths from simple lamp pumped lasers will be very useful in this field by making devices in this field more versatile and cost effective. This invention also offers a method to improve beam quality of flashlamp pumped lasers without adding cost and complexity.

The details of one or more examples are set forth in the accompanying drawings and the description below. Further features, aspects, and advantages of the invention will become apparent from the description, the drawings and the claims.

### BRIEF DESCRIPTION OF THE DRAWINGS

The advantages of the invention described above, together with further advantages, may be better understood by referring to the following description taken in conjunction with the accompanying drawings. The drawings are not necessarily to scale, emphasis instead generally being placed upon illustrating the principles of the invention.
FIG. 1 is a schematic drawing of a solid state laser pumped by a second solid state laser with q-switch and frequency converting elements.
FIG. 2 is a schematic drawing of a handpiece including a solid state laser pumped via an optical fiber by another solid state laser.

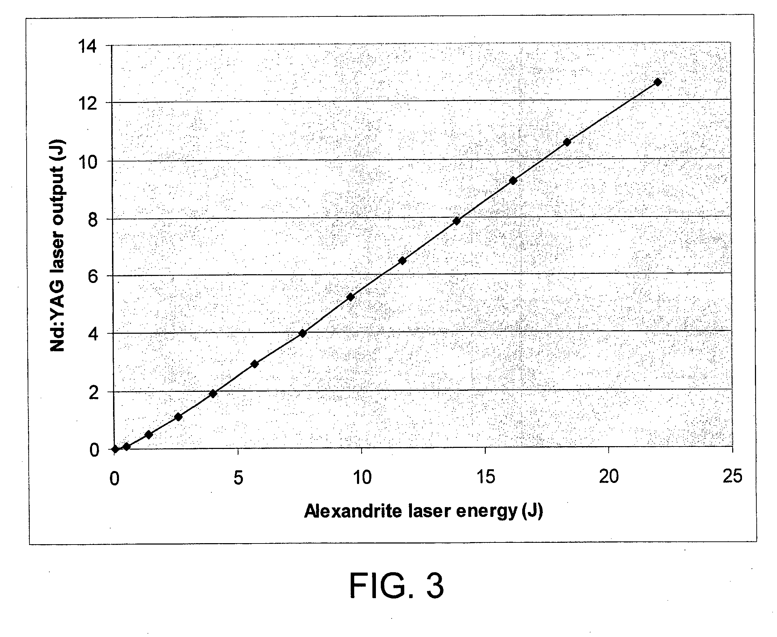

FIG. 3 is a graph of data from an experimental test of a rare-earth laser pumped by a transition metal laser.

### DESCRIPTION OF THE INVENTION

Lasers and other light sources are often used for the treatment of skin disorders and to produce cosmetic improvement in the appearance of the skin including the removal of hair from the skin. The heat produced by the light energy can modify structures within the skin and beneath the skin. Typical applications can include, for example, removal of hair, pigmented lesions, tattoos, vascular lesions, wrinkles, acne, skin tightening, and/or the like. Applications can more generally also include treatment of mammalian tissue.

Dermatological laser treatments can be based on selectively targeting of a chromophore in or near the target structure by an appropriate choice of wavelength and pulse duration of the light. Lasers are often the preferred light source because a laser beam has a narrower wavelength bandwidth than light from other sources. A source with a narrow wavelength bandwidth can maximize the spectral selectivity of the target chromophore. In addition, lasers can be made with much shorter pulse durations than other light sources thereby maximizing the temporal selectivity of the targeted structure. The superior temporal selectivity makes lasers especially preferred for removing small targets like small vessels and tattoo pigment particles.

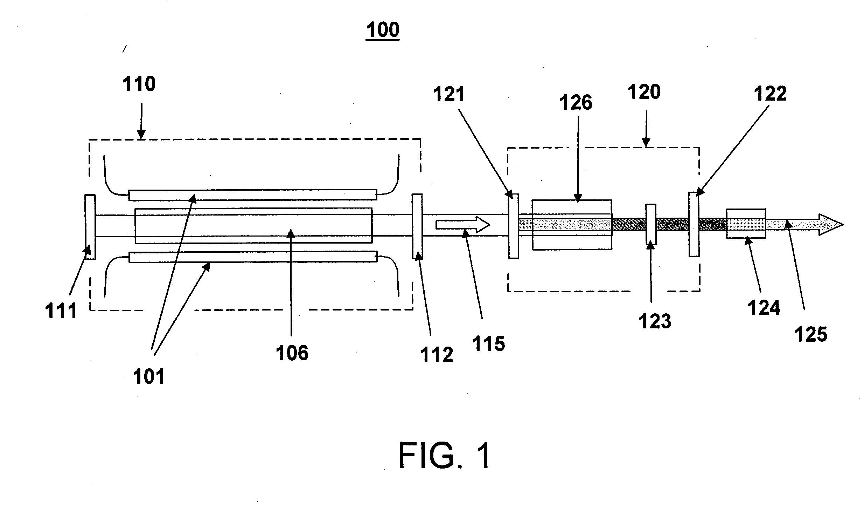

FIG. 1 is a schematic drawing of a laser system **100** including a solid state laser **110** pumping another solid state laser **120.** Laser gain materials **106** and **126** of the solid state lasers **110** and **120,** respectively, can be made in the shape of a round rod, but other configurations can also be used, such as, for example, slabs and cubes. The solid state laser **110** is an Alexandrite laser. The solid state laser **110** can be pumped by one or more flashlamps **101.** The cavity of the solid state laser **110** can include a high reflecting mirror **111** and an output coupler mirror **112.** The output coupler mirror **112** can couple an output beam **115** from the cavity of the first solid state laser **110.** The solid state laser **120** is a rare-earth laser such as, for example, neodymium-YAG (Nd:YAG). The solid state laser **120** is pumped by a portion of the output beam **115.** The output beam **115** can include either long, gain switched pulses, or short, Q-switched, pulses. The temporal profile of the output beam **125** from the laser cavity of the solid state laser **120** has been experimentally found to closely match the temporal profile of the output beam **115** for both long-pulse and/or short-pulse pumping. In addition, a Q-switched solid state laser **110** can be constructed so that long pulses can be produced by not energizing the Q-switching device.

The cavity of the solid state laser **120** can include a high reflecting mirror **121** and an output coupler mirror **122.** The output coupler mirror **122** can couple an output beam **125** from the cavity of the solid state laser **120.** In one embodiment, the cavity of the solid state laser **120** can include a q-switching element **123** such as, for example, a Cr⁴⁺:YAG (Cr:YAG) element. In another embodiment, a frequency doubling crystal such as, for example, KTP, can be positioned in the path of the output beam **125.**

In one embodiment, the solid state laser gain materials **106** and/or **126** can be directly coated on both ends with coatings of appropriate transmission and reflection properties to form the reflecting mirrors **111** and **121** and the output coupling mirrors **112** and **122.** For an Alexandrite/Nd:YAG system, the reflecting mirror **121** and the output coupler mirror **122** can allow both double-pass pumping at about 755 nm and/or laser output at about 1064 nm. The absorption coefficient at about 755 nm can be about 2 cm⁻¹. Therefore, a solid state laser **120** with a gain medium which is 1.15 cm long can absorb approximately 99% of the pump energy in a double-pass configuration.

Coupling the output laser beam **115** of the solid state laser **110** into the gain material **126** of the solid state laser **120** can be accomplished in a variety of ways, such as, for example, end-pumping, as illustrated in FIG. **1**. In one embodiment, the laser gain material **126** of the solid state laser **120** can be made larger than the laser beam **115** so that the solid state laser **120** can be pumped directly without any manipulation of the pump beam **115.** The dimensions of the end of the solid state laser gain material **126** can be made slightly larger than the laser beam **115** so that the solid state laser **120** can be positioned directly in the path of the beam **115.** Alternatively, the beam 115 can be shaped with mirrors, lenses and/or other optical components to optimize the pump volume within the solid state laser **120.** In another embodiment, either solid state laser **110** or solid state laser **120** can be mounted on a translation stage so that the system output beam **125** can be switched between about 755 nm and about 1064 nm simply by moving one of the solid state lasers. Translational stages can be used in systems where output beam **115** and/or output beam **125** are focused into an optical fiber for delivery of the treatment beam. Both output beams **115** and **125** can be more efficiently coupled into the fiber without any positional adjustment of the fiber coupling components (e.g. lens (not shown)).

Both Alexandrite and Nd:YAG lasers can be used in dermatological applications. Therefore, the selection of Alexandrite and Nd:YAG as the solid state lasers **110** and **120,** respectively, in the configuration illustrated in FIG. 1 can allow for complementary applications in a single system. The q-switched versions of both Alexandrite and Nd:YAG lasers, for example, can be used to remove tattoos. Due to the different optical absorption of the various colors of tattoo pigment, an Alexandrite laser can remove blue and green tattoos, while a Nd:YAG laser can remove black tattoos. The second harmonic of a Nd:YAG laser, about 532 nm, can remove red tattoos. Likewise, long-pulse versions of both Alexandrite and Nd:YAG lasers can be effective for hair removal. However, because the wavelengths of Alexandrite and Nd:YAG lasers have different optical absorption in melanin, an Alexandrite laser, at about 755 nm, can be better for treating light-skin patients while a Nd:YAG laser, at about 1064 nm, can be better for dark-skin patients.

The absorption spectra ofNd:YAG has a continuous band of lines ranging from about 725 nm to about 770 nm. Therefore, the non-tuned output of a free-running Alexandrite laser, at about 755 nm, can be used to pump a Nd:YAG laser. An important absorption line in the Nd:YAG is about 2 or 3 nm wide centered at about 755 nm. A stronger but narrower peak is centered at about 750 nm. Furthermore, there are wavelengths within the 725 nm to 770 nm band where excited state absorption can occur. However, there is very little excited state absorption at 755 nm, making it an attractive pump wavelength. The efficiency of the conversion of 755 nm to 1064 nm can be affected mostly by the quantum defect, which is about 30%. There can be another small loss, e.g. less than 5% percent in Nd:YAG, due to scattering effects.

Laser pumping can be particularly attractive for lasers that are difficult to pump with other conventional sources such as, for example, laser diodes and flashlamps. As an example, it can be difficult to generate high peak powers from Nd:YAG at 946 nm, which is one of the laser lines of Nd:YAG. The 946 nm is a three-level laser transition which requires a high pump rate to reach threshold. Flashlamp pumping can be inadequate due to poor brightness of the source, while diode lasers can essentially be continuous wave sources and not suitable for high peak power applications.

When end-pumped by a Q-switched Alexandrite laser emitting a 50 ns pulse at 755 nm, for example, Nd:YAG can readily lase at 946 nm, emitting a similarly short pulse with hundreds of milli-Joules of energy, corresponding to several MW of peak power. In another example, when end-pumped with 25 Joule, 3 millisecond pulses from a free-running, gain-switched Alexandrite laser, an output of 6 Joules at 946 nm can be emitted by the Nd:YAG laser.

Although the pump beam can be absorbed by the gain medium, high absorption is not preferred in all embodiments. For example, some heat can be generated in a rare-earth gain medium by laser pumping, although the amount of heat can be much less than that deposited in the gain medium by flashlamp pumping. Nevertheless, the size of the gain medium can be chosen so that the heat can be removed fast enough to limit the temperature rise in the gain medium. The length of the gain medium and the magnitude of the absorption can be chosen so that the heat generation is distributed fairly evenly through the medium. In some cases, for example, the wavelength emitted by the pump laser can be tuned in order to adjust the absorption of the pump beam by the rare-earth laser.

Absorption spectra show that Nd:YVO₄, and Nd:GdVO₄ can also be excited by a free running Alexandrite laser. A tunable Alexandrite laser, from about 700 nm to about 818 nm, can be used to excite other laser gain materials such as, for example, Nd:YAP, Er:YAG, and/or Tm:YAG. The approximately 2.94 micron wavelength output of a Er:YAG laser has high optical absorption by water and can therefore be used to ablate a thin layer of the epidermis for removing some of the effects of aging and sun damage.

In some embodiments, Tm:YAG can provide laser output when pumped by a free-running Alexandrite laser. For example, when the thulium concentration is at 6 %, a gain length of two inches absorbed 95% of the pump energy with a double-pass pump configuration. The thulium laser can produce 8 Joules of approximately 2 micron laser output when pumped with a 25 Joule, 755 nm laser beam. In this case, about 15 Joules is deposited in the laser rod. The long length of the laser rod can provide sufficient surface area from which to extract the heat between pulses.

Like the Er:YAG above, the approximate 2 micron output of a Tm:YAG laser can be usable for improving the appearance of aged skin. Tm:YAG has the advantage that the wavelength of the output is tunable from about 1.93 microns to about 2.10 microns. The wavelength can be adjusted so that the depth of penetration in the skin can be selected over a range of about 110 micron to about 600 microns.

The laser system **100** can treat a patient at either or both of two wavelengths produced by solid state lasers **110** and **120** at the same or two different pulse durations. For example, a Q-switched Alexandrite laser without a tuning element as solid state laser **110** can produce approximately 50 nanosecond pulses at about 755 nm. The output beam **115** in this configuration can be used to treat the patient or to pump a solid state laser **120** such as Nd:YAG, in which case approximately 50 nanosecond pulses at about 1064 nm can be produced and can be used to treat the patient. In another embodiment, by not energizing or not including a Q-switching element in either cavity of solid state lasers 110 and **120**, the laser system **100** can also be used to treat the patient with long-pulses of either wavelength. In this configuration, the duration of the long pulses can be determined by the duration and output power of the energy pulses produced by the one or more flashlamps **101.**

The laser system **100** can realize one or more of the following advantages over a conventional Q-switched Nd:YAG laser. The duration of the pulse generated by a conventional Q-switched Nd:YAG laser is about 10 nanoseconds. At effective treatment energies, the peak power can be so high that it cannot be transmitted though an optical fiber without damaging the fiber. A conventional Q-switched Nd:YAG laser system, therefore, typically has expensive and inconvenient articulated-arm beam delivery systems to overcome this problem. The 50 nanosecond pulses generated, for example, by the laser system **100** can be transmitted by optical fiber, a simpler and less expensive design. Pulses generated by the laser system 100 can also be as long as 100 nanoseconds. Furthermore, a higher output energy is possible with laser system **100.** Q-switched operation can require that energy be stored in the laser cavity. But amplified spontaneous emission (ASE) can limit the amount of energy that can be stored in a Nd:YAG cavity, resulting in limited output. Gain switched operation in laser system **100,** however, does not have this problem because of the short duration of the pumped state of the laser gain material and of the high Q of the cavity.

In another embodiment, frequency doubling can be used to obtain about a 532 nm output beam **125.** For example, high peak power of 50 nanosecond Nd:YAG pulses from solid state laser **120** can enable efficient second-harmonic conversion of the about 1064 nm wavelength to about 532 nm. Generation of the second-harmonic can be accomplished with a frequency doubling crystal **124,** such as, for example, a KTP crystal. The output laser beam from solid state laser **120** laser can be polarized in order to maximize the efficiency of the wavelength conversion within the frequency doubling crystal **124.** A polarizing element can be installed in the cavity of solid state laser **120. In** an alternative embodiment, a different host material for solid state laser **120** can be selected. Both Nd:YVO₄, and Nd:GdVO₄ can produce linearly polarized outputs at about 1064 nm and about 1063 nm, respectively. The long-pulse 1064 nm beam may not be efficiently frequency doubled because the peak power is low. This problem can be overcome by repetitively Q-switching the solid state laser **120** or the solid-state laser **110.** Either active or passive Q-switching can accomplish repetitive Q-switching. A Cr⁴⁺:YAG passive Q-switch can also be placed in the resonator cavity of solid state laser 120 to generate a train of high peak power pulses that can be efficiently frequency doubled to about 532 nm.

Another method of coupling the pump beam 115 into the solid state laser 120 can include using a fiber optic coupling system. First, one or more lenses or other optical components can converge at least a portion of the pump beam 115 into an optical fiber (not shown) through which a portion of the pump beam 115 can be transmitted. The beam exiting the distal end of the optical fiber can be a divergent beam, which can be directed into the gain material of solid state laser 120 or it can be shaped and/or collimated by one or more lenses or other optical components before being directed into the gain material of solid state laser 120.

In one embodiment, a diode laser output at 808 nm can be used for hair removal and for pumping a Nd: YAG laser. The diode laser system can include an optical system to optimize the divergence of the diode laser beam for treating hair and pumping the Nd: YAG laser.

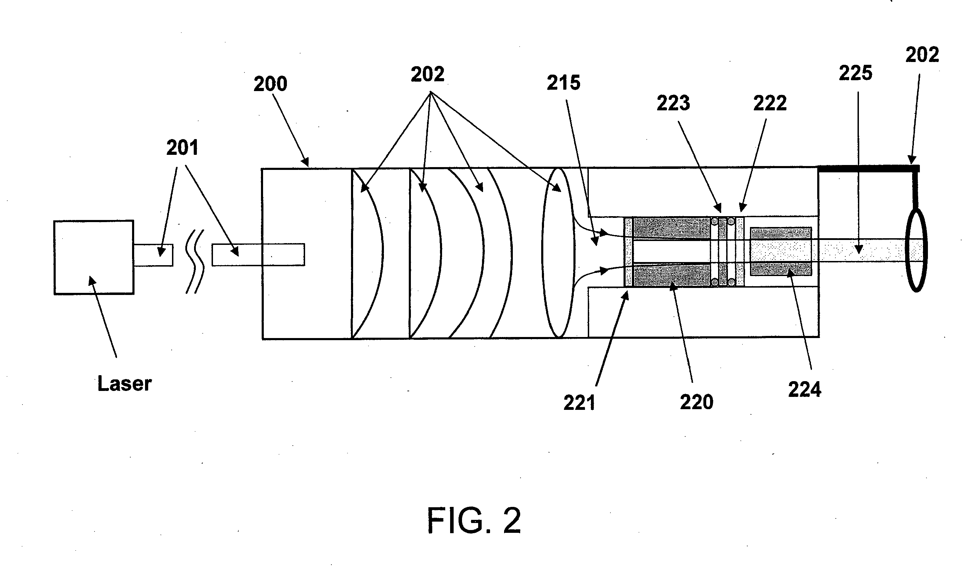

FIG. 2 is a schematic drawing of a handpiece 200 including a solid state laser 220 pumped via an optical fiber 201 by another solid state laser. In various embodiments, the handpiece 200 can include one or more optical components 202 for coupling at least a portion of the output beam 215 from the optical fiber 201 into the cavity of the solid state laser 220. The cavity of the solid state laser 220 can include a high reflecting mirror 221 and an output coupler mirror 222. In one embodiment, the cavity of the solid state laser 220 can include a Q-switching element 223. In another embodiment, the handpiece 200 can include a frequency doubling crystal. The output beam 225 of the handpiece can further be optically modified by an optical component 202 before it is used to treat the skin of a patient. In yet a further embodiment, the solid state laser 220 in the handpiece 200 can include an Er: YAG laser. In another embodiment, the solid state laser 220 in the handpiece 200 can include a Tm:YAG laser with or without a wavelength tuning device such as, for example, a birefringent tuner.

An alexandrite-pumped-neodymium laser system can be useful for a variety of medical applications, in particular dermatology. The three treatment wavelengths and two pulse durations capable of being produced by the laser system 100 can provide a range of six spectrally and temporally selective treatment modes, thereby making this system clinically effective for a large range of medical conditions. The efficient conversion of electrical input energy to laser output energy at all three wavelengths can allow the design of competitively sized and priced laser products. Products based on sub-sets of the elements described herein can also be clinically useful and commercially viable.

To minimize thermal injury to tissue surrounding an eye and/or to an exposed surface of the target region, the delivery system (e.g. handpiece 200) can include a cooling system for cooling before, during and/or after delivery of radiation. Cooling can include contact conduction cooling, evaporative spray cooling, convective air flow cooling, or a combination of the aforementioned. In one embodiment, the handpiece 200 includes a skin contacting portion that can be brought into contact with the skin. The skin contacting portion can include a sapphire or glass window and a fluid passage containing a cooling fluid. The cooling fluid can be a fluorocarbon type cooling fluid, which can be transparent to the radiation used. The cooling fluid can circulate through the fluid passage and past the window to cool the skin.

A spray cooling device can use cryogen, water, or air as a coolant. In one embodiment, a dynamic cooling device can be used to cool the skin (e.g. a DCD available from Candela Corporation). For example, the delivery system can include tubing for delivering a cooling fluid to the handpiece 200. The tubing can be connected to a container of a low boiling point fluid, and the handpiece 200 can include a valve for delivering a spurt of the fluid to the skin. Heat can be extracted from the skin by virtue of evaporative cooling of the low boiling point fluid. The fluid can be a non-toxic substance with high vapor pressure at normal body temperature, such as a Freon or tetrafluoroethane.

The invention has been described in terms of particular embodiments. The alternatives described herein are examples for illustration only and not to limit the alternatives in any way. The steps of the invention can be performed in a different order and still achieve desirable results. Other embodiments are within the scope of the following claims.

## Claims

1. A method of treating skin, comprising the steps of:
generating a first output beam of laser radiation using an alexandrite laser (110);
directing the first output beam to a target region of skin to remove hair or a first tattoo condition from the skin;
generating a second output beam of laser radiation using the alexandrite laser (110);
directing the second output beam to a second solid-state laser (120; 220);
generating a third output beam of laser radiation having a new wavelength using the second solid-state laser (120; 220) based on excitation of a rare-earth doped gain medium by the second output beam; and
directing the third output beam (225) to the target region of skin to remove hair or a second tattoo condition from the skin;
wherein a long-pulsed version of the alexandrite laser is used for non-therapeutic hair removal and a q-switched version of the alexandrite laser is used for tattoo removal.

2. The method of claim 1, wherein the step of directing the first output beam to the target region of skin comprises focusing the first output beam into an optical fiber (201) and a handpiece, (200).

3. The method of claim 1, wherein the step of directing the second output beam to the second solid-state laser (220) comprises focusing the second output beam into an optical fiber (220) and into a first part of a handpiece (200) including the second solid-state laser (220).

4. The method of claim 3, wherein the step of directing the third output beam to the target region of skin comprises directing the third output beam through the handpiece (200).

5. The method of claim 1, wherein the step of removal of the first tattoo condition comprises removing violet tattoos, blue tattoos, green tattoos, black tattoos, or any combination thereof.

6. The method of claim 1, wherein the step of removal of the second tattoo condition comprises removing black tattoos.

7. The method of claim 1, further comprising the steps of:
directing the third output beam to a nonlinear frequency converter (124);
generating a fourth output beam of laser radiation using the nonlinear frequency converter based on the third output beam; and
directing the fourth output beam to the target region of skin to remove a third tattoo condition from the skin.

8. The method of claim 7, wherein the step of directing the second output beam to the second solid-state laser comprises focusing the second output beam into an optical fiber (201) and into a handpiece (200) including the second solid-state laser (220) and a frequency doubling frequency crystal (224).

9. The method of claim 7, wherein the step of removal of the third tattoo condition comprises removing red tattoos, orange tattoos, yellow tattoos, or any combination thereof.

10. The method of claim 1, further comprising using a q-switching element (123) with the alexandrite laser (110) to generate high peak power pulses of the first output beam.

11. The method of claim 1, further comprising the step of :
mounting at least one of the alexandrite laser (110) or the second solid-state laser (120; 220) on a translation stage; and
using the translation stage to move one of the alexandrite laser (110) or the second solid-state laser (120; 220) to switch the third output beam between a wavelength of about 755 nm and about 1064 nm.

12. The method of claim 1, further comprising moving the second solid-state laser (120; 220) to an aligned position in a beam path of the first output beam, wherein the second solid-state laser is neodymium laser configured to absorb at least a portion of the first output beam when in the aligned position.

13. The method of claim 1 further comprising the steps of
forming, using an output coupler mirror, a dual wavelength output beam from the first output beam that passes through the second solid-state laser and laser radiation from the second solid-state laser; and
directing the dual wavelength output beam to the target region to treat the first tattoo condition and the second tattoo condition.

14. A laser system for treating skin, comprising:
a fiber-optic coupling system (201) and an alexandrite laser system (110) for producing a first output beam of laser radiation and configured to direct the first output beam to the fibre-optic (201) coupling system;
a handpiece (200) adapted to be connected to the fibre-optic coupling system (201),
wherein the handpiece (200) comprises a second solid-state laser (220) comprising a rare-earth doped gain medium and adapted to absorb a first portion of the first output beam for generating a second output beam of laser radiation having a new wavelength,
wherein a second portion of the first output beam is focussed into an optical fiber for delivery to a target region to remove hair or a first tattoo condition,
wherein the handpiece (200) is configured to direct the second output beam to the target region to remove a second tattoo condition,
wherein a long-pulsed version of the alexandrite laser is used for hair removal, and a q-switched version of the alexandrite laser is used for tattoo removal.

15. The laser system of claim 14, further comprising a nonlinear frequency converter configured to absorb the second output beam to generate a third output beam (225), wherein the third output beam is configured to remove red tattoos, orange tattoos, yellow tattoos, or any combination thereof.

16. The laser system of claim 14, wherein the alexandrite laser system (120) comprises a q-switching element (123) for generating high peak power pulses of the first output beam.

17. The laser system of claim 14, further comprising a KTP second harmonic generator movable to an aligned position in a beam path of the second output beam to produce a third output beam (225) having a third output wavelength and a beam path coaxial with the beam path of the second output beam.

18. The laser system of claim 14, further comprising at least one coupler mirror (222) configured to form a dual wavelength output beam from the second portion of the first output beam that passes through the handpiece and laser radiation from the handpiece.

## Patentansprüche

1. Verfahren zur Behandlung von Haut, die folgenden Schritte umfassend:
Erzeugen eines ersten Ausgangsstrahls einer Laserstrahlung unter Verwendung eines Alexandrit-Lasers (110);
Lenken des ersten Ausgangsstrahls auf eine Zielregion der Haut, um Haare oder eine erste Tattoo-Bedingung von der Haut zu entfernen;
Erzeugen eines zweiten Ausgangsstrahls einer Laserstrahlung unter Verwendung des Alexandrit-Lasers (110);
Lenken des zweiten Ausgangsstrahls auf einen zweiten Festkörperlaser (120; 220); Erzeugen eines dritten Ausgangsstrahls aus Laserstrahlung mit einer neuen Wellenlänge unter Verwendung des zweiten Festkörperlasers (120; 220) auf Basis einer Anregung eines seltenerddotierten Verstärkungsmediums durch den zweiten Ausgangsstrahl; und
Lenken des dritten Ausgangsstrahls (225) auf die Zielregion der Haut, um Haare oder eine zweite Tattoo-Bedingung von der Haut zu entfernen;
wobei eine langgepulste Version des Alexandrit-Lasers für eine nicht-therapeutische Haarentfernung verwendet wird und eine q-geschaltete Version des Alexandrit-Lasers für eine Tattoo-Entfernung verwendet wird.

2. Verfahren nach Anspruch 1, wobei der Schritt des Lenkens des ersten Ausgangsstrahls auf die Zielregion der Haut das Fokussieren des ersten Ausgangsstrahls in eine optische Faser (201) und ein Handstück (200) umfasst.

3. Verfahren nach Anspruch 1, wobei der Schritt des Lenkens des zweiten Ausgangsstrahls auf den zweiten Festkörperlaser (220) das Fokussieren des zweiten Ausgangsstrahls in eine optische Faser (220) und in einen ersten Teil eines Handstücks (200), welches den zweiten Festkörperlaser (220) aufweist, umfasst.

4. Verfahren nach Anspruch 3, wobei der Schritt des Lenkens des dritten Ausgangsstrahls auf die Zielregion der Haut das Lenken des dritten Ausgangsstrahls durch das Handstück (200) umfasst.

5. Verfahren nach Anspruch 1, wobei der Schritt des Entfernens der ersten Tattoo-Bedingung das Entfernen von violetten Tattoos, blauen Tattoos, grünen Tattoos, schwarzen Tattoos oder irgendeiner Kombination davon aufweist.

6. Verfahren nach Anspruch 1, wobei der Schritt des Entfernens der zweiten Tattoo-Bedingung das Entfernen von schwarzen Tattoos umfasst.

7. Verfahren nach Anspruch 1, ferner die folgenden Schritte umfassend:
Lenken des dritten Ausgangsstrahls auf einen nicht-linearen Frequenzwandler (124);
Erzeugen eines vierten Ausgangsstrahls aus Laserstrahlung unter Verwendung des nicht-linearen Frequenzwandlers auf Basis des dritten Ausgangsstrahls; und
Lenken des vierten Ausgangsstrahls auf die Zielregion der Haut, um eine dritte Tattoo-Bedingung von der Haut zu entfernen.

8. Verfahren nach Anspruch 7, wobei der Schritt des Lenkens des zweiten Ausgangsstrahls auf den zweiten Festkörperlaser das Fokussieren des zweiten Ausgangsstrahls in eine optische Faser (201) und in ein Handstück (200), welches den zweiten Festkörperlaser (220) und einen Frequenzverdopplungskristall (224) aufweist, umfasst.

9. Verfahren nach Anspruch 7, wobei der Schritt des Entfernens der dritten Tattoo-Bedingung das Entfernen von roten Tattoos, orangen Tattoos, gelben Tattoos oder irgendeiner Kombination davon umfasst.

10. Verfahren nach Anspruch 1, ferner das Vewenden eines q-Schalterelements (123) mit dem Alexandrit-Laser (110) umfassend, um Impulse des ersten Ausgangsstrahls mit hoher Spitzenleistung zu erzeugen.

11. Verfahren nach Anspruch 1, ferner die folgenden Schritte umfassend:
Anbauen des Alexandrit-Lasers (110) und/oder des zweiten Festkörperlasers (120; 220) auf einem Verschiebetisch; und
Verwenden des Verschiebetisches, um den Alexandrit-Laser (110) oder den zweiten Festkörperlaser (120; 220) zu bewegen, um den dritten Ausgangsstrahl zwischen einer Wellenlänge von etwa 755 nm und etwa 1064 nm zu schalten.

12. Verfahren nach Anspruch 1, ferner das Bewegen des zweiten Festkörperlasers (120; 220) in eine ausgerichtete Position in einem Strahlenweg des ersten Ausgangsstrahls umfassend, wobei der zweite Festkörperlaser ein Neodymiumlaser ist, der so gestaltet ist, dass er zumindest einen Teil des ersten Ausgangsstrahls absorbiert, wenn er die ausgerichtete Position einnimmt.

13. Verfahren nach Anspruch 1, ferner die folgenden Schritte umfassend:
Ausbilden eines Ausgangsstrahls mit dualer Wellenlänge aus dem ersten Ausgangsstrahl, der durch den zweiten Festkörperlaser geht, und Laserstrahlung aus dem zweiten Festkörperlaser unter Verwendung eines Resonanzspiegels; und
Lenken des Ausgangsstrahls mit der dualen Wellenlänge auf die Zielregion, um die erste Tattoo-Bedingung und die zweite Tattoo-Bedingung zu behandeln.

14. Lasersystem zum Behandeln von Haut, umfassend:
ein faseroptisches Kopplungssystem (201) und ein Alexandrit-Lasersystem (110) zum Erzeugen eines ersten Ausgangsstrahls aus Laserstrahlung, das so gestaltet ist, dass es den ersten Ausgangsstrahl auf dass faseroptische Kopplungssystem (201) lenkt;
ein Handstück (200), das für eine Verbindung mit dem faseroptischen Kopplungssystem (201) ausgelegt ist, wobei das Handstück (200) einen zweiten Festkörperlaser (220) aufweist, der ein seltenerddotiertes Verstärkungsmedium aufweist und dafür ausgelegt ist, einen ersten Teil des ersten Ausgangsstrahls zu absorbieren, um einen zweiten Ausgangsstrahl aus Laserstrahlung mit einer neuen Wellenlänge zu erzeugen,
wobei ein zweiter Abschnitt des ersten Ausgangsstrahls in eine optische Faser fokussiert wird, um an eine Zielregion ausgegeben zu werden, um Haare oder eine erste Tattoo-Bedingung zu entfernen,
wobei das Handstück (200) so gestaltet ist, dass es den zweiten Ausgangsstrahl auf die Zielregion lenkt, um eine zweite Tattoo-Bedingung zu entfernen,
wobei eine langgepulste Version des Alexandrit-Lasers für eine Haarentfernung verwendet wird und eine q-geschaltete Version des Alexandrit-Lasers für eine Tattoo-Entfernung verwendet wird.

15. Lasersystem nach Anspruch 14, ferner einen nicht-linearen Frequenzwandler aufweisend, der so gestaltet ist, dass er den zweiten Ausgangsstrahl absorbiert, um einen dritten Ausgangsstrahl (225) zu erzeugen, wobei der dritte Ausgangsstrahl so gestaltet ist, dass er rote Tattoos, orange Tattoos, gelbe Tattoos oder irgendeine Kombination davon entfernt.

16. Lasersystem nach Anspruch 14, wobei das Alexandrit-Lasersystem (120) ein q-Schalterelement (123) zur Erzeugung von Impulsen des ersten Ausgangsstrahls mit hoher Spitzenleistung aufweist.

17. Lasersystem nach Anspruch 14, ferner einen KTP-Frequenzverdoppler aufweisend, der zu einer ausgerichteten Position in einem Strahlenweg des zweiten Ausgangsstrahls bewegt werden kann, um einen dritten Ausgangsstrahl (225) zu erzeugen, der eine dritte Wellenlänge und einen Strahlenweg aufweist, der koaxial ist zu dem Strahlenweg des zweiten Ausgangsstrahls.

18. Lasersystem nach Anspruch 14, ferner mindestens einen Resonatorspiegel (222) aufweisend, der so gestaltet ist, dass er einen Ausgangsstrahl mit dualer Wellenlänge aus dem zweiten Teil des ersten Ausgangsstrahls, der durch das Handstück geht, und der Laserstrahlung vom Handstück erzeugt.

## Revendications

1. Un procédé de traitement de la peau, comprenant les opérations suivantes :
la génération d'un premier faisceau de sortie de rayonnement laser au moyen d'un laser à alexandrite (110),
la direction du premier faisceau de sortie vers une zone de peau cible de façon à retirer du poil ou un premier état de tatouage de la peau,
la génération d'un deuxième faisceau de sortie de rayonnement laser au moyen du laser à alexandrite (110),
la direction du deuxième faisceau de sortie vers un deuxième laser à solide (120, 220),
la génération d'un troisième faisceau de sortie de rayonnement laser possédant une nouvelle longueur d'onde au moyen du deuxième laser à solide (120, 220) en fonction d'une excitation d'un milieu actif dopé aux terres rares par le deuxième faisceau de sortie, et
la direction du troisième faisceau de sortie (225) vers la zone de peau cible de façon à retirer du poil ou un deuxième état de tatouage de la peau,
où une version à impulsions longues du laser à alexandrite est utilisée pour un retrait de poil non thérapeutique et une version déclenchée du laser à alexandrite est utilisée pour un retrait de tatouage.

2. Le procédé selon la Revendication 1 où l'opération de direction du premier faisceau de sortie vers la zone de peau cible comprend la focalisation du premier faisceau de sortie dans une fibre optique (201) et un instrument à main (200).

3. Le procédé selon la Revendication 1, où l'opération de direction du deuxième faisceau de sortie vers le deuxième laser à solide (220) comprend la focalisation d'un deuxième faisceau de sortie dans une fibre optique (220) et dans une première partie d'un instrument à main (200) comprenant le deuxième laser à solide (220).

4. Le procédé selon la Revendication 3, où l'opération de direction du troisième faisceau de sortie vers la zone de peau cible comprend la direction du troisième faisceau de sortie au travers de l'instrument à main (200).

5. Le procédé selon la Revendication 1, où l'opération de retrait du premier état de tatouage comprend la suppression de tatouages violets, de tatouages bleus, de tatouages verts, de tatouages noirs, ou de toute combinaison de ceux-ci.

6. Le procédé selon la Revendication 1, où l'opération de retrait du deuxième état de tatouage comprend la suppression de tatouages noirs.

7. Le procédé selon la Revendication 1, comprenant en outre les opérations suivantes :
la direction du troisième faisceau de sortie vers un convertisseur de fréquences non linéaire (124),
la génération d'un quatrième faisceau de sortie de rayonnement laser au moyen du convertisseur de fréquences non linéaire en fonction du troisième faisceau de sortie, et
la direction du quatrième faisceau de sortie vers la zone de peau cible de façon à retirer un troisième état de tatouage de la peau.

8. Le procédé selon la Revendication 7, où l'opération de direction du deuxième faisceau de sortie vers le deuxième laser à solide comprend la focalisation du deuxième faisceau de sortie dans une fibre optique (201) et dans un instrument à main (200) comprenant le deuxième laser à solide (220) et un cristal de fréquence à doublage de fréquence (224).

9. Le procédé selon la Revendication 7, où l'opération de retrait du troisième état de tatouage comprend la suppression de tatouages rouges, de tatouages oranges, de tatouages jaunes, ou de toute combinaison de ceux-ci.

10. Le procédé selon la Revendication 1, comprenant en outre l'utilisation d'un élément de modulation de qualité (123) avec le laser à alexandrite (110) de façon à générer des impulsions à puissance de crête élevée du premier faisceau de sortie.

11. Le procédé selon la Revendication 1, comprenant en outre les opérations suivantes :
le montage d'au moins un laser parmi le laser à alexandrite (110) ou le deuxième laser à solide (120, 220) sur un étage de translation, et
l'utilisation de l'étage de translation de façon à déplacer un laser parmi le laser à alexandrite (110) ou le deuxième laser à solide (120, 220) de façon à commuter le troisième faisceau de sortie entre une longueur d'onde d'environ 755 nm et environ 1064 nm.

12. Le procédé selon la Revendication 1, comprenant en outre le déplacement du deuxième laser à solide (120, 220) vers une position alignée dans un trajet de faisceau du premier faisceau de sortie, où le deuxième laser à solide est configuré de façon à absorber au moins une partie du premier faisceau de sortie lorsqu'il se trouve dans la position alignée.

13. Le procédé selon la Revendication 1 comprenant en outre les opérations suivantes :
la formation, au moyen d'un miroir de coupleur de sortie, d'un faisceau de sortie à double longueur d'onde à partir du premier faisceau de sortie qui passe au travers du deuxième laser à solide et d'un rayonnement laser à partir du deuxième laser à solide, et
la direction du faisceau de sortie à double longueur d'onde vers la zone cible de façon à traiter le premier état de tatouage et le deuxième état de tatouage.

14. Un système laser pour un traitement de la peau comprenant :
un système de couplage à fibre optique (201) et un système laser à alexandrite (110) pour la production d'un premier faisceau de sortie de rayonnement laser et configuré de façon à diriger le premier faisceau de sortie vers le système de couplage à fibre optique (201),
un instrument à main (200) adapté de façon à être raccordé au système de couplage à fibre optique (201),
où l'instrument à main (200) comprend un deuxième laser à solide (220) comprenant un milieu actif dopé aux terres rares et adapté de façon à absorber une première partie du premier faisceau de sortie pour la génération d'un deuxième faisceau de sortie de rayonnement laser possédant une nouvelle longueur d'onde,
où une deuxième partie du premier faisceau de sortie est focalisée dans une fibre optique pour une fourniture à une zone cible de façon à retirer du poil ou un premier état de tatouage,
où l'instrument à main (200) est configuré de façon à diriger le deuxième faisceau de sortie vers la zone cible de façon à retirer un deuxième état de tatouage,
où une version à impulsions longues du laser à alexandrite est utilisée pour un retrait de poil et une version déclenchée du laser à alexandrite est utilisée pour un retrait de tatouage.

15. Le système laser selon la Revendication 14, comprenant en outre un convertisseur de fréquences non linéaire configuré de façon à absorber le deuxième faisceau de sortie de façon à générer un troisième faisceau de sortie (225), où le troisième faisceau de sortie est configuré de façon à retirer des tatouages rouges, des tatouages oranges, des tatouages jaunes, ou toute combinaison de ceux-ci.

16. Le système laser selon la Revendication 14, où le système laser à alexandrite (120) comprend un élément de modulation de qualité (123) pour la génération d'impulsions à puissance de crête élevée du premier faisceau de sortie.

17. Le système laser selon la Revendication 14, comprenant en outre un générateur d'harmoniques de deuxième ordre KTP déplaçable vers une position alignée dans un trajet de faisceau du deuxième faisceau de sortie de façon à produire un troisième faisceau de sortie (225) possédant une troisième longueur d'onde de sortie et un trajet de faisceau coaxial avec le trajet de faisceau du deuxième faisceau de sortie.

18. Le système laser selon la Revendication 14, comprenant en outre au moins un miroir de coupleur (222) configuré de façon à former un faisceau de sortie à double longueur d'onde à partir de la deuxième partie du premier faisceau de sortie qui passe au travers de l'instrument à main et un rayonnement laser à partir de l'instrument à main.
